Europäisches Patentamt

European Patent Office

Office européen des brevets

(19)

(11) Publication number: **0 323 710**

**A1**

## (12) EUROPEAN PATENT APPLICATION

(21) Application number: 88311413.4

(22) Date of filing: 01.12.88

(51) Int. Cl.⁴: **C08G 63/60 , C08G 63/68 , C07C 65/24 , C09K 19/38**

(30) Priority: 02.12.87 IT 2285487

(43) Date of publication of application:
**12.07.89 Bulletin 89/28**

(84) Designated Contracting States:
**BE DE ES FR GB NL SE**

(71) Applicant: **Montedison S.p.A.**
**31, Foro Buonaparte**
**I-20121 Milan(IT)**

(72) Inventor: **Gabrielli, Giorgio**
**65, Via Serra**
**I-20149 Milan(IT)**
Inventor: **Maritamo, Mauro**
**77, Via Cavoni**
**I-22069 Rovellasca Como(IT)**
Inventor: **Chapoy, L. Lawrence**
**60 Via Davicini**
**I-28040 Lesa Novara(IT)**
Inventor: **Motroni, Giuseppe**
**5, Via Caduti della Patria**
**I-28066 Galliate Novara(IT)**

(74) Representative: **Lamb, John Baxter et al**
**MARKS & CLERK 57/60 Lincoln's Inn Fields**
**London WC2A 3LS(GB)**

(54) **Thermotropic liquid crystalline aromatic polyesters.**

(57) Thermotropic liquid crystalline aromatic polyesters comprise:

(a) units derived from one or more dicarboxylic aromatic acids of the formula:

$$HOOC\text{-}Ar_1\text{-}O\text{-}Ar_2\text{-}COOH \quad (1)$$

in which:

$Ar_1$ is a divalent aromatic radical consisting of one ring, or of a plurality of condensed, partially condensed or non condensed rings, containing from 6 to 18 carbon atoms, and which may be substituted

$Ar_2$ is a divalent aromatic radical consisting of a plurality of condensed, partially condensed or non-condensed rings, containing from 12 to 18

(b) units derived from one or more aromatic diols of the formula:

$$HO\text{-}Ar_3\text{-}OH \quad (2)$$

in which

$Ar_3$ is a divalent aromatic radical consisting of one ring, or of a plurality of condensed, partially condensed or non-condensed rings, containing from 6 to 18 carbon atoms, and which may be substituted

and if desired, units derived from one or more other aromatic diacids and/or hydroxy acids of the formulae:

(c) $$HOOC\text{-}Ar_3\text{-}COOH \quad (3)$$

(in which $Ar_3$ is as defined above), and

(d) $$HO\text{-}Ar_3\text{-}(CH=CH)_n\text{-}COOH \quad (4)$$

(in which $Ar_3$ is as defined above and $n$ is 0 or 1).

## Thermotropic Liquid Crystalline Aromatic Polyesters

The present invention relates to thermotropic liquid crystalline aromatic polyesters. More particularly, the invention relates to thermotropic liquid crystalline aromatic polyesters which are readily processible in the molten state, and which have a mesogen group in their main chain.

The use of synthetic polymers, both as such and in admixture with reinforcing agents, in the manufacture of articles having good mechanical properties, has rapidly spread during the past ten years. In particular, polyesters have proved to be particularly valuable for preparing injection-moulded or extruded products, such as films or fibres.

A class of polyesters endowed with better than average mechanical properties are those known as thermotropic liquid crystalline polyesters. In general, the thermotropic liquid crystalline polymers show, in the molten state and within a defined temperature range, an ordered arrangement of the molecular chains, which is retained in the solid state, thus giving the polymer desirable properties, such as a high elastic modulus, a high tensile strength and, in general, the typical properties of a fibre-reinforced polymer. Furthermore, these polymers may show a high crystallinity which gives the end products further advantageous properties such as a lower heat distortion and a better resistance to attack by solvents.

Thermotropic liquid crystalline polyesters, which hence show an optical anisotropy in the molten state, are described in the technical literature, such as, e.g., in British Polymer Journal (Dec. 1980), page 154: "Liquid Crystal Polymer"; Journal of Macromolecular Science Chemistry (1984), page 1705: "Liquid Crystalline Aromatic Polyesters"; Die Augewandte Makromolekulare Chemie (1982), 109-110, page 1: "Rigid Chain Polymers"; Die Augewandte Makromolekulare Chemie (1986), 145-46, page 231; "Thermotropic Liquid Crystalline Polymers"; Journal of Molecular Science Review (1986) C26(4), page 551: "Liquid Crystalline Polymers: A Novel State of Material".

The use of such polyesters makes it possible to obtain high tenacity fibres or molded articles from the material in the molten state, by, for example, injection-moulding, which articles have useful characteristics of stiffness, hardness and tenacity.

In accordance with the present invention, a new class of polyesters have been found that have optical anisotropy in the molten state, which can be extruded to yield films or fibres, or moulded to yield end articles, by commonly used methods for processing plastic materials.

According to the invention there are provided thermotropic liquid crystalline aromatic polyesters which comprise:

(a) units derived from one or more aromatic dicarboxylic acids of the formula:

$$HOOC\text{-}Ar_1\text{-}O\text{-}Ar_2\text{-}COOH \qquad (1)$$

in which

$Ar_1$ is a divalent aromatic radical consisting of one ring or a plurality of condensed, partially condensed or non-condensed rings, containing from 6 to 18 carbon atoms, and which may be substituted with halogen atoms, or alkyl, aryl or aralkyl groups containing a small number of carbon atoms; and

$Ar_2$ is a divalent aromatic radical consisting of a plurality of condensed, partially condensed or non-condensed rings, containing from 12 to 18 carbon atoms, and which may be substituted with halogen atoms, or alkyl, aryl or aralkyl groups containing a small number of carbon atoms;

(b) at units derived from one or more aromatic diols of the formula:

$$HO\text{-}Ar_3\text{-}OH \qquad (2)$$

in which:

$Ar^3$ is a divalent aromatic radical consisting of one ring, or of a plurality of condensed, partially condensed or non-condensed rings, containing from 6 to 18 carbon atoms, and which may be substituted with halogen atoms, with alkyl, aryl or aralkyl groups containing a small number of carbon atoms; with cycloalkyl groups containing from 5 to 12 carbon atoms; or with keto groups -COR or ether groups -OR, (in which R is an alkyl group; cycloalkyl group or an aryl group consisting of one ring or of a plurality of condensed, partially condensed or non-condensed rings; containing up to 18 carbon atoms, and optionally being substituted with halogen atoms or with alkyl, aryl or aralkyl group containing a small number of carbon atoms).

and, optionally, units derived from one or more other aromatic diacids and/or hydroxy acids of the formulae:

$$(c) \quad HOOC\text{-}Ar_3\text{-}COOH \qquad (3)$$

(in which $Ar_3$ is as defined above); and

$$(d) \quad HO\text{-}Ar_3O(CH=CH)_n\text{-}COOH \qquad (4)$$

(in which $Ar_3$ is as defined above and $\underline{n}$ is 0 or 1).

By the term "alkyl, aryl or aralkyl groups containing a small number of carbon atoms", as used herein, we mean alkyl groups containing from 1 to 4 carbon atoms and aryl or aralkyl groups containing from 6 to 12 carbon atoms.

According to a preferred embodiment of the invention, at least 80 mol % of the products of formulae (1), (2), (3) and (4) have both their functional groups in such positions as to form a polymeric chain with a substantially coaxial or parallel orientation.

Preferred aromatic dicarboxylic acids of formula (1) are those wherein $Ar_1$ is a 1,4-phenylene; 4,4''-diphenylene, 2,6-naphthylene, or 1,4-naphthylene group; and $Ar_2$ is a 4,4-diphenylene, 2,6-naphthylene or 1,4-naphthylene group.

Examples of aromatic diols of formula (2) are hydroquinone; chloro-bromo-hydroquinone; methyl-, ethyl-, propyl- and tert.-butyl-hydroquinone; phenyl-hydroquinone; (1-phenyl-ethyl)-hydroquinone; (1-methyl-1-phenyl-ethyl)-hydroquinone; cyclohexyl-hydroquinone; benzoyl-hydroquinone; phenoxy-hydroquinone; 4,4'-dihydroxy-diphenyl; 3,3'-dichloro 4,4'dihydroxy-diphenyl; 2,6-dihydroxy-naphthalene; 1,5-dihydroxy-naphthalene; 4,4'-dihydroxy-stilbene; etc.

Examples of aromatic dicarboxylic of formula (3) are terephthalic acid; chloro- and bromo-terephthalic acids; methyl-terephthalic acid; 1,4-naphthalene-dicarboxylic acid; 1,5-naphthalane-dicarboxylic acid; 2,6-naphthalene-dicarboxylic acid; 4,4'diphenylene-dicarboxylic acid; 3,3'dibromo-4,4'-diphenylene-dicarboxylic acid; 4,4'-stilbene-dicarboxylic acid; etc.

Examples of hydroxy acids of formula (4) are p-hydroxy-benzoic acid; 3-chloro-4-hydroxy-benzoic acid; 3-bromo-hydroxy-benzoic acid; 3-tert. butyl-4-hydroxy-benzoic acid; 4-hydroxy-1-naphthoic acid; 6-hydroxy-2-naphthoic acid; p-(4-hydroxy-phenyl) benzoic acid; p-hydroxycinnamic acid; etc.

According to a preferred embodiment of the invention, the units derived from the dicarboxylic acids as defined under (a) and (c) are in molar ratios of (c)/(a) of from 0 to 4, and the units derived from the hydroxy acids as defined under (d) are in olar ratios of (d)/(b), to the aromatic diols as defined under (b), of from 0 to 3. Thermotropic liquid crystalline aromatic polyesters may also be those in which the molar ratios (c)/(a) and (d)/(b) are, respectively, from 0.25 to 2 and from 0.25 to 1.

The polyesters of the inventiona are optically anisotropic in the molten state, as can be verified by means of optical microscopic analysis under polarized light, and have an inherent viscosity, measured in pentafluorophenol at 60° C at a concentration of 0.25 g/litre, of from 0.3 to 4 dl/g.

The melting temperature of the polyesters may vary within wide limits, according to the composition of the polymer and its degree of polymerization. Generally, however, the melting temperature is from 250° to 350° C.

The molecular weight and the crystallinity may be increased by heating the polymer particles in an inert medium, or under vacuum, at a temperature just below the melting point, for a sufficiently long period of time.

The polymers of the invention are suitable for use in the manufacute of fabricated or shaped bodies which may be produced by the conventional techniques for the fabrication of thermoplatic polymers, such as, e.g., by injection moulding or by extrusion. The polymers may be processed to give films or fibres, may be used as matrices for composite materials based on inorganic fibers or fillers, or may be used in blends with other polymers.

The preparation of the polymers according to the invention may be carried out by per se known conventional techniques, by reacting the above mentioned starting materials under normal conditions for the preparation of polyester resins.

The compounds as defined under (b), (c) and (d) are known products and are commercially available or may be readily prepared by well-known techniques. The Compounds of formula (1) are new compounds and may be prepared according to the following reaction shemes:

(A) $HOOC\text{-}Ar_2OOH + X\text{-}Ar_1\text{-}Y \longrightarrow HOOC\text{-}Ar_2\text{-}O\text{-}Ar_1\text{-}Y$;

(B) $HOOC\text{-}Ar_2\text{-}O\text{-}Ar_1\text{-}Y \longrightarrow HOOC\text{-}Ar_2\text{-}O\text{-}Ar_1\text{-}COOH$

wherein:

$Ar_1$ and $Ar_2$ have the meanings as given above;

X is a halogen atom, a nitro group, or any leaving group;

Y is a cyano group, a group $-CON(W)_2$ or $-COOW$ (in which W is $C_1\text{-}C_4$ alkyl), or other electron-attracting group which may be a precursor of the carboxy group.

Reaction (A) is a nucleophilic substitution reaction, generally carried out at a temperature of from 80° to 150° C, and in the presence of an aprotic solvent such as, e.g., dimethylformamide.

Reaction (B) is a normal hydrolysis reaction carried out at a room temperature in an aqueous medium.

The product obtained is recovered from the hydrolysis reaction by precipitation, filtration and drying.

The thermotropic liquid-crystalline polyesters of the invention may be obtained in the molten state, or in the presence of a dispersing medium having a high boiling point (such as diphenyl-sulphone or mixtures of particularly hydrogenated terphenyls) by transesterification between the dicarboxy aromatic acids, and possibly the hydroxy acids, and the acetates or propanoates of the phenols, at temperatures of from 270° to 370° C, so as to favour the complete release of the aliphatic acid. Operation under vaccum is also contemplated.

The reaction may, if desired, be carried out in the presence of a transesterification catalyst such as a phosphate of an alkali or alkaline earth metal. Further catalysts may be those which are commonly used in polycondensation processes, and are described in the "Encyclopaedia of Polymer Science and Technology" (1969, vol. 10, pages 722-723). Examples of such other catalysts are the oxides, hydroxides, hydrides, halides, alkoxides or phenates, the salts and the complex salts of organic or inorganic acids of lithium, sodium, potassium, magnesium, calcium, titanium, manganese, cobalt, zinc, tin, antimony, lathanum, cerium, lead and germanium.

The amount of catalyst is suitably from 0.005 to 1 mol%, and is preferably from 0.01 to 0.2 mol%, calculated on the total amount of the reactants.

According to an alternative method, the polyesters of the invention may be obtained in solution, by polycondensation between the halides of th aromatic dicarboxylic acids and the mixture of phenols in a suitable solvent. The temperature is suitably from 25° to 220°C, and the reaction is carried out in the presence of a base and/or of a stream of nitrogen in order to favour the elimination of the hydrogen halide. Among the bases, pyridine is preferred, whilst among the solvents, there are provided chlorinated solvents, both aliphatic and aromatic, such as methylene chloride, chlorobenzene, dichloro benzenes, and trichloro-benzenes.

The polymer obtained by this second method is subsequently recovered by evaporating off the solvent, or by precipitation with a non-solvent, and subsequent filtration.

In order that the invention may be well understood the following Examples are given by way of illustration only.

## Example 1

### Preparation of 6-(4'carboxy-phenoxy)-2-naphthoic acid

To a 3-neck flask of 1 litre capacity equipped with a mechanical stirrer, reflux condenser and thermometer, the following reactants were charged under a stream of nitrogen:
7.5 g of para-nitro-benzonitrile (50.7 mM);
9.5 g of 6-hydroxy-2-naphthoic acid (53.2 mM);
8.5 g of anhydrous potassium carbonate (85 mM); and
500 ml of anhydrous dimethyl formamide.

The reaction mixture was stirred and heated at 100°C for 6 hours, and was then poured into water (2 litres) and acidified to pH 3 by means of hydrochloric acid.

A brown precipitate was obtained and was recrystallized from acetone/water in a yield of 8.81 g (60%).

This product was hydrolysed in a solution consisting of water (180 ml), methyl-cellosolve (60 ml), and sodium hydroxide (60 g), under refluxing conditions for 4 hours. After the hydrolysis the product was recovered by dilution with water, acidification with HCl, precipitation, filtration, and drying.

The product was purified by recrystallization from acetic acid/$H_2O$, its HPLC purity was higher than 99%, and its structure was confirmed by means of mass-spectrometry. The yield was 82%.

## Example 2

The polymerization equipment employed was a 3-neck flask of 100 ml capacity equipped with a stirrer, nitrogen inlet tube, and distillation head with condenser. The equipment was heated by means of a high-temperature silicone oil bath.

To the above reactor, the following reactants were charged:

(a) 6.00 g of 6-(4'-carboxy-phenoxy)-2-naphthoic acid (19.5 mM);

(b) 4.05 g of 2-methyl-hydroquinone diacetate (19.5 mM);
and

(c) 0.060 g of tribasic sodium phosphate dodecahydrate.

Under a slight nitrogen stream, the reaction mixture was heated for 30 minutes at 200°C, then for 30 minutes at 240°C, for 30 minutes at 260°C, for 1 hour at 280°C, for 1 hour at 300°C, and finally for a further 1 hour at 300°C under a vacuum of 0.5-1 millibars. During the whole process, the distilled acetic acid has removed from the reaction.

The reaction mass, after cooling to room temperature, was recovered, weighed and finely ground.

The polymer, which was in the form of a powder of a light beige colour, had an inherent viscosity (at 0.25% in pentafluorophenol at 60°C) of 0.5 dl/g, and, when subjected to DSC (Differential Scanning Calorimetry) analysis, showed an endothermic peak at 280°C, which was repeated during subsequent cooling/heating cycles. In the cooling cycle (20°C/minute), an exothermic peak at 250°C was detected, which indicated a quick crystallization.

The polymer, when examined under polarized light on a microscope equipped with a heated stage, showed, at temperatures above 280°C, strong light birefringence, correlated with the anisotropic nature of the polymer in the molten state.

## Example 3

In the same equipment and following the same procedures as in Example 2, the following reactants were polymerized:

(a) 6.00 g of 6-(4'-carboxy-phenoxy)-2-naphthoic acid (19.5 mM);

(b) 4.47 g of 2-chloro-hydroquinone diacetate (19.5 mM);
and

(c) 0.060 g of tribasic sodium phosphate dodecahydrate.

The polymer obtained had an inherent viscosity of 0.51 dl/g and, when subjected to DSC analysis, showed a melting point of 267° C, and a crystallization temperature of 246° C.

The molten polymer was optically anisotropic when examined under the polarized-light microscope.

Example 4

In the same equipment and following the same procedures as in Example 2, the following reactants were polymerized:

(a) 6.00 g of 6-(4'-carboxy-phenoxy)-2-naphthoic acid (19.5 mM);

(b) 1.89 g of hydroquinone diacetate (9.75 mM);

(c) 2.03 g of 2-methyl-hydroquinone diacetate (9.75 mM);
and

(d) 0.01 g of tribasic sodium phosphate dodecahydrate.

The polymer thus obtained had an intrinsic viscosity of 1.12 dl/g and, when subjected to DSC analysis, showed an endothermic peak at 356° C, which was repeated during the subsequent cooling/heating cycles. In the cooling cycle, an exothermic peak was evidenced at 316° C, showing a quick crystallization. When observed under the polarized-light microscope, the polymer was birefringent in the molten state.

**Claims**

1. A thermotropic liquid-crystalline aromatic polyester characterized in that it comprises:
(a) units derived from one or more dicarboxylic aromatic acids of the formula:
$$HOOC-Ar_1-O-Ar_2-COOH \qquad (1)$$
in which:
$Ar_1$ is a divalent aromatic radical consisting of one ring, or of a plurality of condensed, partially condensed or non condensed rings, containing from 6 to 18 carbon atoms, and which may be substituted with halogen atom, or alkyl, aryl oraralkyl groups containing a small number of carbon atoms; and
$Ar_2$ is a divalent aromatic radical consisting of a plurality of codensed, partially condensed or non-condensed rings, containing from 12 to 18 carbon atoms, and which may be substituted with halogen atoms, or alkyl, aryl or aralkyl groups containing a small number of carbon atoms;
(b) units derived from one or more aromatic diols of the formula:
$$HO-Ar_3-OH \qquad (2)$$
in which
$Ar_3$ is a divalent aromatic radical consisting of one ring, or of a plurality of condensed, partially condensed or non-condensed rings, containing from 6 to 18 carbon atoms, and which may be substituted with halogen atoms or with alkyl, aryl or aralkyl groups containing a small number of carbon atoms, with cycloalkyl groups containing from 5 to 12 carbon atoms, or with keto groups -COR or ether groups -OR, (wherein R is an alkyl group, or cycloalkyl group, an aryl group consisting of one ring or of a plurality of condensed, partially condensed or non-condensed rings; contains from 1 to 18 carbon atoms; and may be substituted with halogen atoms or with alkyl, aryl or aralkyl groups containing a small number of carbon atoms);
and if desired, units derived from one or more other aromatic diacids and/or hydroxy acids of the formulae:
(c) $\qquad HOOC-Ar_3-COOH \qquad (3)$
( in which $Ar_3$ is as defined above), and
(d) $\qquad HO-Ar_3-(CH=CH)_n-COOH \qquad (4)$
(in which $Ar_3$ is as defined above and $\underline{n}$ is 0 or 1).

2. Polyesters according to claim 1, characterised in that at least 80 mol % of the products of formulae (1), (2), (3) and (4) have their two functional groups in such positions as to form a polymeric chain with a substantially coaxial or parallel direction.

3. Polyesters according to claim 1 or claim 2 characterised in that dicarboxylic aromatic acid of formula (1) is one in which $Ar_1$ is a 1,4-phenylene, 4,4'-diphenylene or 2,6-naphthylene group; and $Ar_2$ is a 4,4'-diphenylene; 2,6-naphthylene or 1,4-naphthylene group.

4. Polyesters according to any one of the preceding claims characterised in that the aromatic diol is hydroquinone; chloro or bromo-hydroquinone; methyl-, ethyl-, propyl or tert.-butyl-hydroquinone; phenyl-hydroquinone; (1 phenyl-ethyl)-hydroquinone; (1-methyl- 1-phenyl-ethyl)-hydroquinone; cyclohexyl-hydroquinone; benzoyl-hydroquinone; phenoxy-hydroquinone; 4-4'dihydroxy-diphenyl; 3,3'-dibromo-4,4'dihydroxy-diphenyl; 3,3'-dichloro-4,4'-dihydroxy-diphenyl; 3,3'-dimethyl-4,4'-dihydroxy-diphenyl; 3,3'-diphenyl-4,4'-

dihydroxy-diphenyl; 2,6-dihydroxy-naphthalene; 1,5-dihydroxy-naphthalene; or 4,4'-dihydroxy-stilbene.

5. Polyesters acording to any one of the preceding claims characterised in that the aromatic diacid of formula (3) is terephthalic acid; chloro- or bromo-terephthalic acid; methyl- terephthalic acid; 1,4-naphthalene-dicarboxylic acid; 1-5 naphthalene-dicarboxylic acid; 2,6-naphthalene-dicarboxylic acid; 4,4'diphenylene-dicarboxylic acid; 3,3'-dibromo-4,4'-diphenylene-dicarboxylic acid; 4,4'-stilbene-dicarboxy acid; and/or the hydroxy acid is p-hydroxy-benzoic acid; 3-chloro-4-hydroxy-benzoic acid; 3-bromo-4-hydroxy-benzoic acid; 3,5-dichloro-4-hydroxy-benzoic acid; 3-methyl-4-hydroxy-benzoic acid; 3-tert.-butyl-4-hydroxy-benzoic acid; 4-hydroxy-1-naphthoic acid; 6-hydroxy-2-naphthoic acid; p-(4-hydroxy-phenyl)-benzoic acid; or p-hydroxycinnamic acid.

6. Polyesters according to any one of the preceeding claims characterised in that the molar ratio, (c)/(a), of the units derived from the dicarboxylic acids as defined under (a) and (c) is from 0 to 4, and the molar rato (d)/(b) of the units derived from the hydroxy acids as defined under (d) to the aromatic diols as defined under (b), is from 0 to 3.

7. Polyesters according to claim 7 characterised in that the molar ratios (c)/(a) and (d)/(b) are, respectively, from 0.25 to 2 and from 0.25 to 1.

8. Polyesters according to any one of the preceeding claims characterised in that they have an inherent viscosity, measured in pentafluorophenol at 60°C at a concentration of 0.25 g/liter, of from 0.3 to 4 dl/g, and a melting temperature of from 250° to 350°C.

9. Polyesters as claimed in any of the preceeding claims in the form of fibres, films or shaped bodies fromed by injection or extrusion; as matrices for composite materials based on inorganic fibres or fillers; or of mixtures with other polymes.

10. Aromatic dicarboxylic acids of the formula:
HOOC-Ar₁-O-Ar₂-COOH
in which Ar₁ and Ar₂ have the meanings defined in claim 3.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | PATENT ABSTRACTS OF JAPAN vol. 11, no. 297 (C-448)(2744) 25th September 1987; & JP - A - 62 89641 (TEIJIN LTD) 24-04-1987 * whole abstract * | 1,10 | C 08 G 63/60 C 08 G 63/68 C 07 C 65/24 C 09 K 19/38 |
| A | EP-A-0 022 344 (CELANESE CORPORATION) * claims 1,5,6; page 3, lines 16-22 * | 1,4,5,9 | |
| A | EP-A-0 083 426 (E.I. DU PONT DE NEMOURS AND COMPANY) * claims 1,2; page 16, lines 15-18 * | 1,9 | |
| A | THE BRITISH POLYMER JOURNAL December 1980, pages 132-146; J.-I. JIN et al.: "Thermotropic Liquid Crystalline Polyesters with Rigid or Flexible Spacer Groups" * page 134, line 36; page 135; table 2 * | 1 | |

TECHNICAL FIELDS SEARCHED (Int. Cl.4)

C 08 G
C 09 K
C 07 C

The present search report has been drawn up for all claims

| Place of search | Date of completion of the search | Examiner |
|---|---|---|
| BERLIN | 28-02-1989 | VOIGTLAENDER R O J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)